# EUROPÄISCHE PATENTANMELDUNG

(11) **EP 1 106 693 A1**
(43) Veröffentlichungstag der Anmeldung: **13.06.2001**
(21) Anmeldenummer: 00125832.6
(22) Anmeldetag: 25.11.2000
(51) Int. Cl.: C12N 15/52, C12N 9/00, C12N 15/77, C12N 1/20, C12P 13/08, C12Q 1/68

(54) **Für das zwa2-Protein codierende Nukleotidsequenzen**

(30) Priorität: 09.12.1999 DE 19959327
(71) Anmelder: Degussa AG, 40474 Düsseldorf (DE)
(72) Erfinder: Möckel, Bettina, Dr., 40597 Düsseldorf (DE); Weissenborn, Anke, 72076 Tübingen (DE); Pfefferle, Walter, Dr., 33790 Halle (Westf.) (DE); Marx, Achim, Dr., 33613 Bielefeld (DE); Pühler, Alfred, Prof., 33793 Bielefeld (DE); Kalinowski, Jörn, Dr., 33615 Bielefeld (DE); Bathe, Brigitte, Dr., 33154 Salzkotten (DE); Dusch, Nicole, Dr., 33619 Bielefeld (DE)

(57) **Zusammenfassung**

Die Erfindung betrifft neue isolierte Polynukleotide, enthaltend eine Polynukleotidsequenz, ausgewählt aus der Gruppe
a) Polynukleotid, das mindestens zu 70 % identisch ist mit einem Polynukleotid, das für ein Polypeptid codiert, das die Aminosäuresequenz von SEQ ID NO 2
b) Polynukleotid, das für ein Polypeptid codiert, das eine Aminosäuresequenz enthält, die zu mindestens 70 % identisch ist mit der Aminosäuresequenz von SEQ ID NO 2 bzw.
c) Polynukleotid, das komplementär ist zu den Polynukleotiden von a) und b) und
d) Polynukleotid, enthaltend mindestens 15 aufeinanderfolgende Nukleotide der Polynukleotidsequenz von a), b) oder c)
und Verfahren zur fermentativen Herstellung von L-Aminosäure unter Abschwächung des zwa2-Gens in den eingesetzten coryneformen Bakterien.

## Beschreibung

Gegenstand der Erfindung sind die für das zwa2-Gen kodierende Nukleotidsequenzen und ein Verfahren zur fermentativen Herstellung von Aminosäuren, insbesondere L-Lysin, unter Verwendung von coryneformen Bakterien, in denen das zwa2-Gen abgeschwächt wird.

### Stand der Technik

Aminosäuren, insbesondere L-Lysin, finden in der Humanmedizin und in der pharmazeutischen Industrie, insbesondere aber in der Tierernährung Anwendung.

Es ist bekannt, daß Aminosäuren durch Fermentation von Stämmen coryneformer Bakterien, insbesondere Corynebacterium glutamicum, hergestellt werden. Wegen der großen Bedeutung wird ständig an der Verbesserung der Herstellverfahren gearbeitet. Verfahrensbesserungen können fermentationstechnische Maßnahmen wie z. B. Rührung und Versorgung mit Sauerstoff, oder die Zusammensetzung der Nährmedien wie z. B. die Zuckerkonzentration während der Fermentation, oder die Aufarbeitung zur Produktform durch z. B. Ionenaustauschchromatographie oder die intrinsischen Leistungseigenschaften des Mikroorganismus selbst betreffen.

Zur Verbesserung der Leistungseigenschaften dieser Mikroorganismen werden Methoden der Mutagenese, Selektion und Mutantenauswahl angewendet. Auf diese Weise erhält man Stämme, die resistent gegen Antimetabolite wie z. B. das Lysin-Analogon S-(2-Aminoethyl)-Cystein oder auxotroph für regulatorisch bedeutsame Metabolite sind und L-Aminosäuren produzieren.

Seit einigen Jahren werden ebenfalls Methoden der rekombinanten DNA-Technik zur Stammverbesserung Aminosäureproduzierender Stämme von Corynebacterium eingesetzt.

### Aufgabe der Erfindung

Die Erfinder haben es sich zur Aufgabe gestellt, neue Maßnahmen zur verbesserten fermentativen Herstellung von Aminosäuren, insbesondere L-Lysin, bereitzustellen.

### Beschreibung der Erfindung

L-Aminosäuren, insbesondere L-Lysin, finden in der Humanmedizin, in der pharmazeutischen Industrie und insbesondere in der Tierernährung Anwendung. Es besteht daher ein allgemeines Interesse daran, neue verbesserte Verfahren zur Herstellung von Aminosäuren, insbesondere L-Lysin, bereitzustellen.

Wenn im folgenden L-Lysin oder Lysin erwähnt werden, sind damit nicht nur die Base, sondern auch die Salze wie z. B. Lysin-Monohydrochlorid oder Lysin-Sulfat gemeint.

Gegenstand der Erfindung ist ein isoliertes Polynukleotid aus coryneformen Bakterien, enthaltend eine Polynukleotidsequenz, ausgewählt aus der Gruppe
a) Polynukleotid, das zu mindestens 70 % identisch ist mit einem Polynukleotid, das für ein Polypeptid codiert, das die Aminosäuresequenz von SEQ ID NO 2 enthält,
b) Polynukleotid, das für ein Polypeptid codiert, das eine Aminosäuresequenz enthält, die zu mindestens 70% identisch ist mit der Aminosäuresequenz von SEQ ID NO 2,
c) Polynukleotid, das komplementär ist zu den Polynukleotiden von a) oder b), und
d) Polynukleotid, enthaltend mindestens 15 aufeinanderfolgende Nukleotide der Polynukleotidsequenz von a), b) oder c).

Gegenstand der Erfindung ist ebenfalls ein Polynukleotid gemäß Anspruch 1, wobei es sich bevorzugt um eine replizierbare DNA handelt, enthaltend:
(i) die Nukleotidsequenz, gezeigt in SEQ ID NO 1 , die für das zwa2-Gen codiert,
(ii) mindestens eine Sequenz, die der Sequenz (i) innerhalb des Bereichs der Degeneration des genetischen Codes entspricht, oder
(iii) mindestens eine Sequenz, die mit zu den Sequenzen (i) oder (ii) komplementären Sequenzen hybridisiert, und gegebenenfalls
(iv) funktionsneutralen Sinnmutationen in (i).

Weitere Gegenstände sind
ein Polynukleotid gemäß Anspruch 4, enthaltend die Nukleotidsequenz wie in SEQ ID NO 1 dargestellt,
ein Vektor, enthaltend das Polynukleotid gemäß Anspruch 1, Punkt d, insbesondere pCR2.1zwa2int, hinterlegt in E.coli DSM 13113
und als Wirtszelle dienende coryneforme Bakterien, die durch Integrationsmutagenese mit dem Vektor gemäß Anspruch 6 enthalten werden.

Gegenstand der Erfindung sind ebenso Polynukleotide, die im wesentlichen aus einer Polynukleotidsequenz bestehen, die erhältlich sind durch Screening mittels Hybridisierung einer entsprechenden Genbank, die das vollständige Gen mit der Polynukleotidsequenz entsprechend SEQ ID NO 1 oder Teile davon enthält, mit einer Sonde, die die Sequenz des genannten Polynukleotids gemäß SEQ ID NO 1 oder ein Fragment davon enthält und Isolierung der genannten DNA-Sequenz.

Polynukleotidsequenzen gemäß der Erfindung sind als Hybridisierungs-Sonden für RNA, cDNA und DNA geeignet, um cDNA in voller Länge zu isolieren, die für das Zwa2-Genprodukt codieren und um solche cDNA oder Gene zu isolieren, die eine hohe Ähnlichkeit der Sequenz mit der des zwa2-Gens aufweisen.

Polynukleotidsequenzen gemäß der Erfindung sind weiterhin als Primer geeignet, mit deren Hilfe durch die Polymerase-Kettenreaktion (PCR) DNA von Genen hergestellt werden kann, die für das zwa2-Gen codieren.

Solche als Sonden oder Primer dienende Oligonukleotide enthalten mindestens 30, bevorzugt mindestens 20, ganz besonders bevorzugt mindestens 15 aufeinanderfolgende Nukleotiden. Geeignet sind ebenfalls Oligonukleotide mit einer Länge von mindestens 40 oder 50 Nukleotiden.

"Isoliert" bedeutet aus seinem natürlichen Umfeld herausgetrennt.

"Polynukleotid" bezieht sich im allgemeinen auf Polyribonukleotide und Polydeoxyribonukleotide, wobei es sich um nicht modifizierte RNA oder DNA oder modifizierte RNA oder DNA handeln kann.

Unter "Polypeptiden" versteht man Peptide oder Proteine, die zwei oder mehr über Peptidbindungen verbundene Aminosäuren enthalten.

Die Polypeptide gemäß Erfindung schließen Polypeptide gemäß SEQ ID NO 2, insbesondere solche mit der biologischen Aktivität des Genproduktes des zwa2-Gens und auch solche ein, die wenigstens zu 70 % identisch sind mit dem Polypeptid gemäß SEQ ID NO 2, bevorzugt wenigstens zu 80% und besonders die wenigstens zu 90 % bis 95 % Identität mit dem Polypeptid gemäß SEQ ID NO 2 und die genannte Aktivität aufweisen.

Die Erfindung betrifft weiterhin ein Verfahren zur fermentativen Herstellung von Aminosäuren, insbesondere L-Lysin, unter Verwendung von coryneformen Bakterien, die insbesondere bereits die Aminosäure produzieren und in denen die für das zwa2-Gen codierenden Nukleotidsequenzen abgeschwächt, insbesondere auf niedrigem Niveau exprimiert werden.

Die Mikroorganismen, die Gegenstand der vorliegenden Erfindung sind, können L-Lysin aus Glucose, Saccharose, Lactose, Fructose, Maltose, Melasse, Stärke, Cellulose oder aus Glycerin und Ethanol herstellen. Es kann sich um Vertreter coryneformer Bakterien insbesondere der Gattung Corynebacterium handeln. Bei der Gattung Corynebacterium ist insbesondere die Art Corynebacterium glutamicum zu nennen, die in der Fachwelt für ihre Fähigkeit bekannt ist, L-Aminosäuren zu produzieren.

Geeignete Stämme der Gattung Corynebacterium, insbesondere der Art Corynebacterium glutamicum, sind zum Beispiel die bekannten Wildtypstämme
Corynebacterium glutamicum ATCC13032
Corynebacterium acetoglutamicum ATCC15806
Corynebacterium acetoacidophilum ATCC13870
Corynebacterium melassecoloa ATCC17965
Corynebacterium thermoaminogenes FERM BP-1539
Brevibacterium flavum ATCC14067
Brevibacterium lactofermentum ATCC13869 und
Brevibacterium divaricatum ATCC14020
und daraus hergestellte L-Lysin produzierende Mutanten bzw. Stämme, wie beispielsweise
Corynebacterium glutamicum FERM-P 1709
Brevibacterium flavum FERM-P 1708
Brevibacterium lactofermentum FERM-P 1712
Corynebacterium glutamicum FERM-P 6463
Corynebacterium glutamicum FERM-P 6464 und
Corynebacterium glutamicum DSM5715

Den Erfindern gelang es, das neue, für das Zwa2-Genprodukt codierende zwa2-Gen von C. glutamicum zu isolieren.

Zur Isolierung des zwa2-Gens oder auch anderer Gene von C. glutamicum wird zunächst eine Genbank dieses Mikroorganismus in E. coli angelegt. Das Anlegen von Genbanken ist in allgemein bekannten Lehrbüchern und Handbüchern niedergeschrieben. Als Beispiel seien das Lehrbuch von Winnacker: Gene und Klone, Eine Einführung in die Gentechnologie (Verlag Chemie, Weinheim, Deutschland, 1990) oder das Handbuch von Sambrook et al.: Molecular Cloning, A Laboratory Manual (Cold Spring Harbor Laboratory Press, 1989) genannt. Eine sehr bekannte Genbank ist die des E. coli K-12 Stammes W3110, die von Kohara et al. (Cell 50, 495 - 508 (1987)) in λ-Vektoren angelegt wurde. Bathe et al. (Molecular and General Genetics, 252:255-265, 1996) beschreiben eine Genbank von C. glutamicum ATCC13032, die mit Hilfe des Cosmidvektors SuperCos I (Wahl et al., 1987, Proceedings of the National Academy of Sciences USA, 84:2160-2164) im E. coli K-12 Stamm NM554 (Raleigh et al., 1988, Nucleic Acids Research 16:1563-1575) angelegt wurde. Börmann et al. (Molecular Microbiology 6(3), 317-326 (1992)) wiederum beschreiben eine Genbank von C. glutamicum ATCC13032 unter Verwendung des Cosmids pHC79 (Hohn und Collins, Gene 11, 291-298 (1980)). Zur Herstellung einer Genbank von C. glutamicum in E. coli können auch Plasmide wie pBR322 (Bolivar, Life Sciences, 25, 807-818 (1979)) oder pUC9 (Vieira et al., 1982, Gene, 19:259-268) verwendet werden. Als Wirte eignen sich besonders solche E. coli-Stämme, die restriktions- und rekombinationsdefekt sind. Ein Beispiel hierfür ist der Stamm DH5amcr, der von Grant et al. (Proceedings of the National Academy of Sciences USA, 87 (1990) 4645-4649) beschrieben wurde. Die mit Hilfe von Cosmiden klonierten langen DNA-Fragmente können anschließend wiederum in gängige für die Sequenzierung geeignete Vektoren subkloniert und anschließend sequenziert werden, so wie es z. B. bei Sanger et al. (Proceedings of the National Academy of Sciences of the United States of America, 74:5463-5467, 1977) beschrieben ist.

Auf diese Weise wurde die neue, für das zwa2-Gen kodierende DNA-Sequenz von C. glutamicum erhalten, die als SEQ ID NO 1 Bestandteil der vorliegenden Erfindung ist. Weiterhin wurde aus der vorliegenden DNA-Sequenz die Aminosäuresequenz des entsprechenden Genproduktes des zwa2-Gens abgeleitet. In SEQ ID NO 2 ist die sich ergebende Aminosäuresequenz des Zwa2-Genproduktes dargestellt.

Kodierende DNA-Sequenzen, die sich aus den SEQ ID NO 1 durch die Degeneriertheit des genetischen Codes ergeben, sind ebenfalls Bestandteil der Erfindung. In gleicher Weise sind DNA-Sequenzen, die mit der SEQ ID NO 1 oder Teilen von SEQ ID NO 1 hybridisieren Bestandteil der Erfindung. Schließlich sind DNA-Sequenzen Bestandteil der Erfindung, die durch die Polymerase-Kettenreaktion (PCR) unter Verwendung von Primern hergestellt werden, die sich aus SEQ ID No. 1 ergeben.

Anleitungen zur Identifizierung von DNA-Sequenzen mittels Hybridisierung findet der Fachmann unter anderem im Handbuch "The DIG System Users Guide for Filter Hybridization" der Firma Boehringer Mannheim GmbH (Mannheim, Deutschland, 1993) und bei Liebl et al. (International Journal of Systematic Bacteriology (1991) 41: 255-260). Anleitungen zur Amplifikation von DNA-Sequenzen mit Hilfe der Polymerase-Kettenreaktion (PCR) findet der Fachmann unter anderem im Handbuch von Gait: Oligonukleotide synthesis: a practical approach (IRL Press, Oxford, UK, 1984) und bei Newton und Graham: PCR (Spektrum Akademischer Verlag, Heidelberg, Deutschland, 1994).

Die Erfinder fanden heraus, daß coryneforme Bakterien nach Abschwächung des zwa2-Gens in verbesserter Weise Aminosäuren, insbesondere L-Lysin, produzieren.

Zur Erzielung einer Abschwächung können entweder die Expression des zwa2-Gens oder die katalytischen Eigenschaften des Enzymproteins herabgesetzt oder ausgeschaltet werden. Gegebenenfalls können beide Maßnahmen kombiniert werden.

Die Erniedrigung der Genexpression kann durch geeignete Kulturführung oder durch genetische Veränderung (Mutation) der Signalstrukturen der Genexpression erfolgen. Signalstrukturen der Genexpression sind beispielsweise Repressorgene, Aktivatorgene, Operatoren, Promotoren, Attenuatoren, Ribosomenbindungsstellen, das Startkodon und Terminatoren. Angaben hierzu findet der Fachmann z. B. in der Patentanmeldung WO 96/15246, bei Boyd und Murphy (Journal of Bacteriology 170: 5949 (1988)), bei Voskuil und Chambliss (Nucleic Acids Research 26: 3548 (1998), bei Jensen und Hammer (Biotechnology and Bioengineering 58: 191 (1998)), bei Pätek et al. (Microbiology 142: 1297 (1996)) und in bekannten Lehrbüchern der Genetik und Molekularbiologie wie z. B. dem Lehrbuch von Knippers ("Molekulare Genetik", 6. Auflage, Georg Thieme Verlag, Stuttgart, Deutschland, 1995) oder dem von Winnacker ("Gene und Klone", VCH Verlagsgesellschaft, Weinheim, Deutschland, 1990).

Mutationen, die zu einer Veränderung bzw. Herabsetzung der katalytischen Eigenschaften von Enzymproteinen führen, sind aus dem Stand der Technik bekannt; als Beispiele seien die Arbeiten von Qiu und Goodman (Journal of Biological Chemistry 272: 8611-8617 (1997)), Sugimoto et al. (Bioscience Biotechnology and Biochemistry 61: 1760-1762 (1997)) und Möckel ("Die Threonindehydratase aus Corynebacterium glutamicum: Aufhebung der allosterischen Regulation und Struktur des Enzyms", Berichte des Forschungszentrums Jülichs,Jül-2906, ISSN09442952, Jülich, Deutschland, 1994) genannt. Zusammenfassende Darstellungen können bekannten Lehrbüchern der Genetik und Molekularbiologie wie z. B. dem von Hagemann ("Allgemeine Genetik", Gustav Fischer Verlag, Stuttgart, 1986) entnommen werden.

Als Mutationen kommen Transitionen, Transversionen, Insertionen und Deletionen in Betracht. In Abhängigkeit von der Wirkung des Aminosäureaustausches auf die Enzymaktivität wird von Fehlsinnmutationen (missense mutations) oder Nichtsinnmutationen (nonsense mutations) gesprochen. Insertionen oder Deletionen von mindestens einem Basenpaar in einem Gen führen zu Rasterverschiebungsmutationen (frame shift mutations), in deren Folge falsche Aminosäuren eingebaut werden oder die Translation vorzeitig abbricht. Deletionen von mehreren Kodonen führen typischerweise zu einem vollständigen Ausfall der Enzymaktivität. Anleitungen zur Erzeugung derartiger Mutationen gehören zum Stand der Technik und können bekannten Lehrbüchern der Genetik und Molekularbiologie wie z. B. dem Lehrbuch von Knippers ("Molekulare Genetik", 6. Auflage, Georg Thieme Verlag, Stuttgart, Deutschland, 1995), dem von Winnacker ("Gene und Klone", VCH Verlagsgesellschaft, Weinheim, Deutschland, 1990) oder dem von Hagemann ("Allgemeine Genetik", Gustav Fischer Verlag, Stuttgart, 1986) entnommen werden.

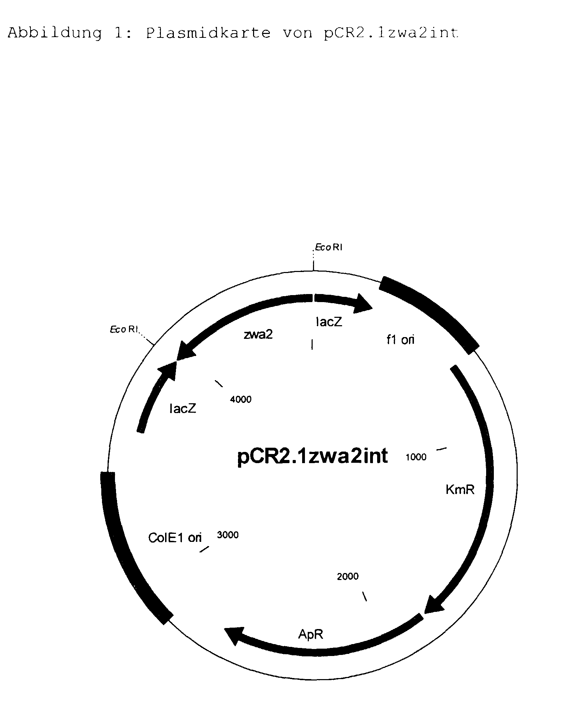

Ein Beispiel für ein Plasmid, mit Hilfe dessen eine Insertionsmutagenese des zwa2-Gens durchgeführt werden kann, ist pCR2.1zwa2int (Figur 1).

Plasmid pCR2.1zwa2int besteht aus dem von Mead at al. (Bio/Technology 9:657-663 (1991)) beschriebenen Plasmid pCR2.1-TOPO, in das ein internes Fragment des zwa2-Gens, dargestellt in SEQ ID No. 3, eingebaut wurde. Dieses Plasmid führt nach Transformation und homologer Rekombination in das chromosomale zwa2-Gen (Insertion) zu einem Totalverlust der Funktion. Auf diese Weise wurde beispielhaft der Stamm DSM5715::pCR2.1zwa2int hergestellt, dessen zwa2-Gen ausgeschaltet ist. Weitere Anleitungen und Erläuterungen zur Insertionsmutagenese findet man beispielsweise bei Schwarzer und Pühler (Bio/Technology 9,84-87 (1991)) oder Fitzpatrick et al. (Applied Microbiology and Biotechnology 42, 575-580 (1994)).

Zusätzlich kann es für die Produktion von L-Aminosäuren insbesondere L-Lysin vorteilhaft sein, zusätzlich zur Abschwächung des zwa2-Gens eines oder mehrere Enzyme des jeweiligen Biosyntheseweges, der Glykolyse, der Anaplerotik, des Zitronensäure-Zyklus oder des Aminosäure-Exports zu verstärken, insbesondere zu überexprimieren.

So kann beispielsweise für die Herstellung von L-Lysin eines oder mehrere der Gene, ausgewählt aus der Gruppe
- das für die Dihydrodipicolinat-Synthase kodierende dapA-Gen (EP-B 0 197 335),
- das für die Tetradihydrodipicolinat Succinylase kodierende dapD Gen (Wehrmann et al., Journal of Bacteriology 180, 3159-3165 (1998)),
- das für eine feed back resistente Aspartatkinase kodierende lysC-Gen,
- das Gen für die Succinyldiaminopimelate-Desuccinylase kodierende dapE Gen (Wehrmann et al., Journal of Bacteriology 177: 5991-5993 (1995)),
- das für die Glyceraldehyd-3-Phosphat Dehydrogenase kodierende gap-Gen (Eikmanns (1992), Journal of Bacteriology 174:6076-6086),
- das für die Pyruvat Carboxylase codierende pyc-Gen (DE-A-198 31 609),
- das für die Malat:Chinon Oxidoreduktase kodierende mqo-Gen (Molenaar et al., European Journal of Biochemistry 254, 395 - 403 (1998)),
- das für den Lysin-Export kodierende lysE-Gen (DE-A-195 48 222)
gleichzeitig verstärkt, insbesondere überexprimiert oder amplifiziert werden.

Weiterhin kann es für die Produktion von Aminosäuren, insbesondere L-Lysin vorteilhaft sein, neben dem zwa2-Gen gleichzeitig
- das für die Phosphatpyruvat-Carboxykinase codierende Gen (DE 199 50 409.1; DSM 13047) und/oder
- das für die Glucose-6-Phosphat Isomerase kodierende pgi-Gen (US 09/396,478; DSM 12969)
abzuschwächen.

Weiterhin kann es für die Produktion von Aminosäuren, insbesondere L-Lysin, vorteilhaft sein, neben der Abschwächung des zwa2-Gens unerwünschte Nebenreaktionen auszuschalten (Nakayama: "Breeding of Amino Acid Producing Micro-organisms", in: Overproduction of Microbial Products, Krumphanzl, Sikyta, Vanek (eds.), Academic Press, London, UK, 1982).

Die das Polynukleotid gemäß Anspruch 1 enthaltenden Mikroorganismen sind ebenfalls Gegenstand der Erfindung und können kontinuierlich oder diskontinuierlich im batch - Verfahren (Satzkultivierung) oder im fed batch (Zulaufverfahren) oder repeated fed batch Verfahren (repetitives Zulaufverfahren) zum Zwecke der Produktion von Aminosäuren insbesondere L-Lysin kultiviert werden. Eine Zusammenfassung über bekannte Kultivierungsmethoden sind im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) beschrieben.

Das zu verwendende Kulturmedium muß in geeigneter Weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D.C., USA, 1981) enthalten. Als Kohlenstoffquelle können Zucker und Kohlehydrate wie z.B. Glucose, Saccharose, Lactose, Fructose, Maltose, Melasse, Stärke und Cellulose, Öle und Fette wie z. B. Sojaöl, Sonnenblumenöl, Erdnussöl und Kokosfett, Fettsäuren wie z. B. Palmitinsäure, Stearinsäure und Linolsäure, Alkohole wie z. B. Glycerin und Ethanol und organische Säuren wie z. B. Essigsäure verwendet werden. Diese Stoffe können einzeln oder als Mischung verwendet werden. Als Stickstoffquelle können organische Stickstoffhaltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden. Als Phosphorquelle können Phosphorsäure, Kaliumdihydrogenphosphat oder Dikaliumhydrogenphosphat oder die entsprechenden Natrium-haltigen Salze verwendet werden. Das Kulturmedium muß weiterhin Salze von Metallen enthalten wie z.B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Dem Kulturmedium können überdies geeignete Vorstufen zugesetzt werden. Die genannten Einsatzstoffe können zur Kultur in Form eines einmaligen Ansatzes hinzugegeben oder in geeigneter Weise während der Kultivierung zugefüttert werden.

Zur pH-Kontrolle der Kultur werden basische Verbindungen wie Natriumhydroxid, Kaliumhydroxid, Ammoniak bzw. Ammoniakwasser oder saure Verbindungen wie Phosphorsäure oder Schwefelsäure in geeigneter Weise eingesetzt. Zur Kontrolle der Schaumentwicklung können Antischaummittel wie z. B. Fettsäurepolyglykolester eingesetzt werden. Zur Aufrechterhaltung der Stabilität von Plasmiden können dem Medium geeignete selektiv wirkende Stoffe wie z. B. Antibiotika hinzugefügt werden. Um aerobe Bedingungen aufrechtzuerhalten werden Sauerstoff oder Sauerstoffhaltige Gasmischungen wie z. B. Luft in die Kultur eingetragen. Die Temperatur der Kultur liegt normalerweise bei 20°C bis 45°C und vorzugsweise bei 25°C bis 40°C. Die Kultur wird solange fortgesetzt bis sich ein Maximum an Lysin gebildet hat. Dieses Ziel wird normalerweise innerhalb von 10 Stunden bis 160 Stunden erreicht.

Methoden zur Bestimmung von L-Aminosäuren sind aus dem Stand der Technik bekannt. Die Analyse kann so wie bei Spackman et al. (Analytical Chemistry, 30, (1958), 1190) beschrieben durch Anionenaustauschchromatographie mit anschließender Ninhydrin Derivatisierung erfolgen, oder sie kann durch reversed phase HPLC erfolgen, so wie bei Lindroth et al. (Analytical Chemistry (1979) 51: 1167-1174) beschrieben.

Ein für die Mutagenese geeigneter Integrationsvektor wurde in E.coli bei der Deutschen Sammlung für Mikrorganismen und Zellkulturen (DSMZ, Braunschweig, Deutschland) gemäß Budapester Vertrag hinterlegt:
- Escherichia coli Stamm TOP10F'/pCR2.1zwa2int als DSM 13113

Zusätzlich zur Abschwächung des zwa2-Gens kann es vorteilhaft sein, das zwa1-Gen oder die Wirkung des zugehörigen Zwa1-Genprodukts zu verstärken. Das entsprechende Gen und die zugehörigen Maßnahmen finden sich in der parallel eingereichten deutschen Patentanmeldung 199 59 328.0.

Ein für die Mutagenese geeigneter Integrationsvektor pCR2.1zwa1exp wurde unter der Nr. DSM13115 in E.coli DH5α hinterlegt.

### Beispiele

Die vorliegende Erfindung wird im folgenden anhand von Ausführungsbeispielen näher erläutert.

### Beispiel 1

### Herstellung einer genomischen Cosmid-Genbank aus Corynebacterium glutamicum ATCC 13032

Chromosomale DNA aus Corynebacterium glutamicum ATCC 13032 wurde wie bei Tauch et al. (1995, Plasmid 33:168-179) beschrieben, isoliert und mit dem Restriktionsenzym Sau3AI (Amersham Pharmacia, Freiburg, Deutschland, Produktbeschreibung Sau3AI, Code no. 27-0913-02) partiell gespalten. Die DNA-Fragmente wurden mit shrimp alkalischer Phosphatase (Roche Molecular Biochemicals, Mannheim, Deutschland, Produktbeschreibung SAP, Code no. 1758250) dephosphoryliert. Die DNA des Cosmid-Vektors SuperCosl (Wahl et al. (1987) Proceedings of the National Academy of Sciences USA 84:2160-2164), bezogen von der Firma Stratagene (La Jolla, USA, Produktbeschreibung SuperCosl Cosmid Vektor Kit, Code no. 251301) wurde mit dem Restriktionsenzym XbaI (Amersham Pharmacia, Freiburg, Deutschland, Produktbeschreibung XbaI, Code no. 27-0948-02) gespalten und ebenfalls mit shrimp alkalischer Phosphatase dephosphoryliert. Anschließend wurde die Cosmid-DNA mit dem Restriktionsenzym BamHI (Amersham Pharmacia, Freiburg, Deutschland, Produktbeschreibung BamHI, Code no. 27-0868-04) gespalten. Die auf diese Weise behandelte Cosmid-DNA wurde mit der behandelten ATCC13032-DNA gemischt und der Ansatz mit T4-DNA-Ligase (Amersham Pharmacia, Freiburg, Deutschland, Produktbeschreibung T4-DNA-Ligase, Code no.27-0870-04) behandelt. Das Ligationsgemisch wurde anschließend mit Hilfe des Gigapack II XL Packing Extracts (Stratagene, La Jolla, USA, Produktbeschreibung Gigapack II XL Packing Extract, Code no. 200217) in Phagen verpackt. Zur Infektion des E. coli Stammes NM554 (Raleigh et al. 1988, Nucleic Acid Research 16:1563-1575) wurden die Zellen in 10 mM MgSO₄ aufgenommen und mit einem Aliquot der Phagensuspension vermischt. Infektion und Titerung der Cosmidbank wurden wie bei Sambrook et al. (1989, Molecular Cloning: A laboratory Manual, Cold Spring Harbor) beschrieben durchgeführt, wobei die Zellen auf LB-Agar (Lennox, 1955, Virology, 1:190) mit 100 µg/ml Ampicillin ausplattiert wurden. Nach Inkubation über Nacht bei 37°C wurden rekombinante Einzelklone selektioniert.

### Beispiel 2

### Isolierung und Sequenzierung des zwa2-Gens

Die Cosmid-DNA einer Einzelkolonie wurde mit dem Qiaprep Spin Miniprep Kit (Product No. 27106, Qiagen, Hilden, Germany) nach Herstellerangaben isoliert und mit dem Restriktionsenzym Sau3AI (Amersham Pharmacia, Freiburg, Deutschland, Produktbeschreibung Sau3AI, Product No. 27-0913-02) partiell gespalten. Die DNA-Fragmente wurden mit shrimp alkalischer Phosphatase (Roche Molecular Biochemicals, Mannheim, Deutschland, Produktbeschreibung SAP, Product No. 1758250) dephosphoryliert. Nach gelelektrophoretischer Auftrennung erfolgte die Isolierung der Cosmidfragmente im Größenbereich von 1500 bis 2000 bp mit dem QiaExII Gel Extraction Kit (Product No. 20021, Qiagen, Hilden, Germany). Die DNA des Sequenziervektors pZero-1 bezogen von der Firma Invitrogen (Groningen, Niederlande, Produktbeschreibung Zero Background Cloning Kit, Product No. K2500-01) wurde mit dem Restriktionsenzym BamHI (Amersham Pharmacia, Freiburg, Deutschland, Produktbeschreibung BamHI, Product No. 27-0868-04) gespalten. Die Ligation der Cosmidfragmente in den Sequenziervektor pZero-1 wurde wie von Sambrook et al. (1989, Molecular Cloning: A laboratory Manual, Cold Spring Harbor) beschrieben durchgeführt, wobei das DNA-Gemisch mit T4-Ligase (Pharmacia Biotech, Freiburg, Deutschland) über Nacht inkubiert wurde. Dieses Ligationsgemisch wurde anschließend in den E. coli Stamm DH5αMCR (Grant, 1990, Proceedings of the National Academy of Sciences U.S.A., 87:4645-4649) elektroporiert (Tauch et al. 1994, FEMS Microbiol Letters, 123:343-7) und auf LB-Agar (Lennox, 1955, Virology, 1:190) mit 50 µg/ml Zeocin ausplattiert. Die Plasmidpräparation der rekombinanten Klone erfolgte mit dem Biorobot 9600 (Product No. 900200, Qiagen, Hilden, Deutschland). Die Sequenzierung erfolgte nach der Dideoxy-Kettenabbruch-Methode von Sanger et al. (1977, Proceedings of the National Academy of Sciences U.S.A., 74:5463-5467) mit Modifikationen nach Zimmermann et al. (1990, Nucleic Acids Research, 18:1067). Es wurde der "RR dRhodamin Terminator Cycle Sequencing Kit" von PE Applied Biosystems(Product No. 403044, Weiterstadt, Deutschland) verwendet. Die gelelektrophoretische Auftrennung und Analyse der Sequenzierreaktion erfolgte in einem "Rotiphorese NF Acrylamid/Bisacrylamid" Gel (29:1) (Product No. A124.1, Roth, Karlsruhe, Germany) mit dem "ABI Prism 377" Sequenziergerät von PE Applied Biosystems (Weiterstadt, Deutschland).

Die erhaltenen Roh-Sequenzdaten wurden anschließend unter Anwendung des Staden-Programpakets (1986, Nucleic Acids Research, 14:217-231) Version 97-0 prozessiert. Die Einzelsequenzen der pZerol-Derivate wurden zu einem zusammenhängenden Contig assembliert. Die computergestützte Kodierbereichsanalyse wurden mit dem Programm XNIP (Staden, 1986, Nucleic Acids Research, 14:217-231) angefertigt. Homologieanalysen wurden mit den "BLAST search programs" (Altschul et al., 1997, Nucleic Acids Research, 25:3389-3402) gegen die non-redundant Datenbank des "National Center for Biotechnology Information" (NCBI, Bethesda, MD, USA) durchgeführt.

Die erhaltene Nukleotidsequenz des zwa2-Gens ist in SEQ ID NO 1 dargelegt. Die Analyse der Nukleotidsequenz ergab ein offenes Leseraster von 1740 Basenpaaren, welches als zwa2-Gen bezeichnet wurde. Das zwa2-Gen kodiert für ein Polypeptid von 385 Aminosäuren, welches in SEQ ID NO 2 dargelegt ist.

### Beispiel 3

### Herstellung eines Integrationsvektors für die Insertionsmutagenese des zwa2-Gens

Aus dem Stamm ATCC 13032 wurde nach der Methode von Eikmanns et al. (Microbiology 140: 1817-1828 (1994)) chromosomale DNA isoliert. Aufgrund der aus Beispiel 2 für C. glutamicum bekannten Sequenz des zwa2-Gens wurden die folgenden Oligonukleotide für die Polymerase Kettenreaktion ausgewählt:

zwa2-in1:
5` GGA ACT TGG TGA CCA GGA CA 3`

zwa2-in2:
5` CTG GCT TTG CTG CGG TGA TT 3`

Die dargestellten Primer wurden von der Firma MWG Biotech (Ebersberg, Deutschland) synthetisiert und nach der Standard-PCR-Methode von Innis et al. (PCR protocols. A guide to methods and applications, 1990, Academic Press) mit Pwo-Polymerase der Firma Boehringer die PCR Reaktion durchgeführt. Mit Hilfe der Polymerase-Kettenreaktion wurde ein ca. 0,6 kb großes DNA-Fragment, dargestellt in SEQ ID No. 3, isoliert, welches ein internes Fragment des zwa2-Gens beinhaltet.

Das amplifizierte DNA Fragment wurde mit dem TOPO TA Cloning Kit der Firma Invitrogen Corporation (Carlsbad, CA, USA; Katalog Nummer K4500-01) in den Vektor pCR2.1-TOPO (Mead at al. (1991) Bio/Technology 9:657-663) ligiert. Anschließend wurde der E. coli Stamm Top10F' mit dem Ligationsansatz (Hanahan, In: DNA cloning. A practical approach. Vol.I. IRL-Press, Oxford, Washington DC, USA) elektroporiert. Die Selektion von Plasmid-tragenden Zellen erfolgte durch Ausplattieren des Transformationsansatzes auf LB Agar (Sambrook et al., Molecular cloning: a laboratory manual. 2^{nd} Ed. Cold Spring Harbor Laboratory Press, Cold Spring Harbor, N.Y.), der mit 25 mg/l Kanamycin supplementiert worden war. Plasmid-DNA wurde aus einer Transformante mit Hilfe des QIAprep Spin Miniprep Kit der Firma Qiagen isoliert und durch Restriktion mit dem Restriktionsenzym EcoRI und anschließender Agarosegel-Elektrophorese (0,8%) überprüft. Das Plasmid wurde pCR2.1zwa2int genannt.

### Beispiel 4

### Integrationsmutagenese des zwa2-Gens in dem Lysinproduzenten DSM 5715

Der in Beispiel 2 genannte Vektor pCR2.1zwa2int wurde nach der Elektroporationsmethode von Tauch et al.(FEMS Microbiological Letters, 123:343-347 (1994)) in Corynebacterium glutamicum DSM 5715 elektroporiert. Bei dem Stamm DSM 5715 handelt es sich um einen AEC resistenten Lysin-Produzenten. Der Vektor pCR2.1zwa2int kann in DSM5715 nicht selbständig replizieren und bleibt nur dann in der Zelle erhalten, wenn er ins Chromosom von DSM 5715 integriert hat. Die Selektion von Klonen mit ins Chromosom integriertem pCR2.1zwa2int erfolgte durch Ausplattieren des Elektroporationsansatzes auf LB Agar (Sambrook et al., Molecular cloning: a laboratory manual. 2^{nd} Ed. Cold Spring Harbor Laboratory Press, Cold Spring Harbor, N.Y., 1989), der mit 15 mg/l Kanamycin supplementiert worden war. Für den Nachweis der Integration wurden nach der Standardmethode von Innis et al. (PCR protocols. A guide to methods and applications, 1990, Academic Press) mit Pwo-Polymerase der Firma Boehringer Kontroll-PCR Reaktionen durchgeführt. Durch Kombination der Primer zwa1-in1 und zwa2-in2 (vgl. Beispiel 3) mit den Primern M13 universal forward (5'-gttttcccagtcacgac-3'; Invitrogen Corporation, Cat. No. N540-02) und M13 universal reverse (5'-caggaaacagctatgac-3'; Invitrogen Corporation, Cat. No. N530-02), die nur innerhalb der Sequenz des Vektors pCR2.1zwa2int binden können, konnte gezeigt werden, daß das Plasmid pCR2.1zwa2int innerhalb des chromosomalen zwa2 Gens in das Chromosom des Lysinproduzenten DSM5715 inseriert hatte. Der Stamm wurde als DSM5715::pCR2.1zwa2int bezeichnet.

### Beispiel 5

### Herstellung von Lysin

Der in Beispiel 3 erhaltene C. glutamicum Stamm DSM5715::pCR2.1zwa2int wurde in einem zur Produktion von Lysin geeigneten Nährmedium kultiviert und der Lysingehalt im Kulturüberstand bestimmt.

Dazu wurde der Stamm zunächst auf Agarplatte mit dem entsprechenden Antibiotikum (Hirn-Herz Agar mit Kanamycin (25 mg/l) für 24 Stunden bei 33°C inkubiert. Ausgehend von dieser Agarplattenkultur wurde eine Vorkultur angeimpft (10 ml Medium im 100 ml Erlenmeyerkolben). Als Medium für die Vorkultur wurde das Vollmedium CgIII verwendet. Diesem wurde Kanamycin (25 mg/l) zugesetzt. Die Vorkultur wurde 48 Stunden bei 33°C bei 240 rpm auf dem Schüttler inkubiert. Von dieser Vorkultur wurde eine Hauptkultur angeimpft, so daß die Anfangs-OD (660 nm) der Hauptkultur 0,1 betrug. Für die Hauptkultur wurde das Medium MM verwendet.

| Medium MM | |
|---|---|
| CSL (Corn Steep Liquor) | 5 g/l |
| MOPS | 20 g/l |
| Glucose(getrennt autoklaviert) | 50g/l |

| Salze: | |
|---|---|
| (NH₄)₂SO₄ | 25 g/l |
| KH₂PO₄ | 0,1 g/l |
| MgSO₄ * 7 H₂O | 1,0 g/l |
| CaCl₂ * 2 H₂O | 10 mg/l |
| FeSO₄ * 7 H₂O | 10 mg/l |
| MnSO₄ * H₂O | 5,0mg/l |
| Biotin (sterilfiltriert) | 0,3 mg/l |
| Thiamin * HCl (sterilfiltriert) | 0,2 mg/l |
| Leucin (sterilfiltriert) | 0,1 g/l |
| CaCO₃ | 25 g/l |

CSL, MOPS und die Salzlösung werden mit Ammoniakwasser auf pH 7 eingestellt und autoklaviert. Anschließend werden die sterilen Substrat- und Vitaminlösungen zugesetzt, sowie das trocken autoklavierte CaCO₃.

Die Kultivierung erfolgt in 10 ml Volumen in einem 100 ml Erlenmeyerkolben mit Schikanen. Es wurde Kanamycin (25 mg/l) zugesetzt. Die Kultivierung erfolgte bei 33°C und 80% Luftfeuchte.

Nach 48 Stunden wurde die OD bei einer Meßwellenlänge von 660 nm mit dem Biomek 1000 (Beckmann Instruments GmbH, München) ermittelt. Die gebildete Lysinmenge wurde mit einem Aminosäureanalysator der Firma Eppendorf-BioTronik (Hamburg, Deutschland) durch Ionenaustauschchromatographie und Nachsäulenderivatisierung mit Ninhydrindetektion bestimmt.

In Tabelle 1 ist das Ergebnis des Versuchs dargestellt.

**Tabelle 1**

| Stamm | OD(660) | Lysin-HCl g/l |
|---|---|---|
| DSM5715::pCR2.1zwa2int | 12,7 | 12,29 |
| DSM5715 | 13,1 | 9,54 |

SEQUENZPROTOKOLL

<110> Degussa-Hüls AG

<120> Neue für das zwa2-Gen codierende Nucleotidsequenzen

<130> 990153 BT

<140>
<141>

<160> 3

<170> PatentIn Ver. 2.1

<210> 1
<211> 1740
<212> DNA
<213> Corynebacterium glutamicum

<220>
<221> CDS
<222> (341)..(1495)

<400> 1
gtattgcgcc gatttcccag attttgattg aaaccgatgc gccgtatatg acgccggagc 60

cgtttcgggg gagtaggaat gagccgtcgt tgattggtca tacggcgcta tgcattgcgg 120

aggttcgggg gatggctgtg gaggatgttg cggcggcttt gaatgagaat tttgatcgcg 180

tttatggggt cacaaatcta taacgtgagg tagctcacag tcaatctgtt ggccgtggtc 240

agctgtgggg gttgtggtgg gtgtgactga agtttatgaa gttgcacgcc acggcgtttt 300

ggtgatggac gggggtagtt tgttaccgta ttgtgactaa ttg tta att ccc ccg 355
Leu Leu Ile Pro Pro
1               5

aga gcg aag aag ttt tac atg gcg ccc cat cag aag tca cgg atc aac 403
Arg Ala Lys Lys Phe Tyr Met Ala Pro His Gln Lys Ser Arg Ile Asn
                10                  15                  20

cgg atc aac agc acc cgc tcg gtg ccg ttg cgt ttg gct acc ggt ggc 451
Arg Ile Asn Ser Thr Arg Ser Val Pro Leu Arg Leu Ala Thr Gly Gly
            25                  30                  35

gtg ctc gcc acc ttg ctt atc ggc gga gtc acc gct gca gct acc aaa 499
Val Leu Ala Thr Leu Leu Ile Gly Gly Val Thr Ala Ala Ala Thr Lys
        40                  45                  50

aag gac atc att gtt gat gtc aac ggc gag cag atg tcc cta gtg act 547
Lys Asp Ile Ile Val Asp Val Asn Gly Glu Gln Met Ser Leu Val Thr
    55                  60                  65

atg tcc ggc act gtt gaa ggt gtg ctg gcg caa gct ggt gtg gaa ctt 595
Met Ser Gly Thr Val Glu Gly Val Leu Ala Gln Ala Gly Val Glu Leu
70                  75                  80                  85

ggt gac cag gac att gtt tcc cct tca ctg gat tca tcc atc agt gat 643
Gly Asp Gln Asp Ile Val Ser Pro Ser Leu Asp Ser Ser Ile Ser Asp
                90                  95                  100

gaa gac act gtg act gtt cgt act gcc aag cag gtg gcg ctc gtg gtg 691
Glu Asp Thr Val Thr Val Arg Thr Ala Lys Gln Val Ala Leu Val Val
            105                 110                 115

gaa ggt caa atc caa aac gtg acc acc act gcg gtt tcc gtg gag gac 739
Glu Gly Gln Ile Gln Asn Val Thr Thr Thr Ala Val Ser Val Glu Asp
        120                 125                 130

ctc ctg cag gaa gtc ggt ggc att acc ggt gct gat gcg gtg gac gct 787
Leu Leu Gln Glu Val Gly Gly Ile Thr Gly Ala Asp Ala Val Asp Ala
    135                 140                 145

gat ctt tca gag acc atc cca gaa tct ggt ttg aag gtg agt gtt acc 835
Asp Leu Ser Glu Thr Ile Pro Glu Ser Gly Leu Lys Val Ser Val Thr
150                 155                 160                 165

aag ccg aag att att tcc atc aat gat ggt ggc aag gtc act tac gtt 883
Lys Pro Lys Ile Ile Ser Ile Asn Asp Gly Gly Lys Val Thr Tyr Val
                170                 175                 180

tct ttg gca gct cag aac gta cag gaa gcc cta gag ctg cgg gat att 931
Ser Leu Ala Ala Gln Asn Val Gln Glu Ala Leu Glu Leu Arg Asp Ile
            185                 190                 195

gag ctg ggt gct cag gac cgc att aat gtg cct ctg gat cag cag ctg 979
Glu Leu Gly Ala Gln Asp Arg Ile Asn Val Pro Leu Asp Gln Gln Leu
        200                 205                 210

aag aac aac gct gcg atc cag atc gac cgc gtt gac aac acc gaa atc 1027
Lys Asn Asn Ala Ala Ile Gln Ile Asp Arg Val Asp Asn Thr Glu Ile
    215                 220                 225

act gaa act gtg tct ttc gat gct gag cca acc tac gtg gat gat cca 1075
Thr Glu Thr Val Ser Phe Asp Ala Glu Pro Thr Tyr Val Asp Asp Pro
230                 235                 240                 245

gaa gct cca gct ggc gat gaa act gtg gtc gaa gaa ggc gct cct gga 1123
Glu Ala Pro Ala Gly Asp Glu Thr Val Val Glu Glu Gly Ala Pro Gly
                250                 255                 260

acc aag gaa gtt act cgc acc gta aca acc gtt aat ggt cag gaa gaa 1171
Thr Lys Glu Val Thr Arg Thr Val Thr Thr Val Asn Gly Gln Glu Glu
            265                 270                 275

tct tcc acg gtg atc aat gaa gtt gaa atc acc gca gca aag cca gca 1219
Ser Ser Thr Val Ile Asn Glu Val Glu Ile Thr Ala Ala Lys Pro Ala
        280                 285                 290

acc att agc cgt ggc acc aaa act gtc gct gca aac tcc gtg tgg gat 1267
Thr Ile Ser Arg Gly Thr Lys Thr Val Ala Ala Asn Ser Val Trp Asp
    295                 300                 305

cag ctg gca cag tgt gaa tcc ggc gga aac tgg gca atc aac aca ggt 1315
Gln Leu Ala Gln Cys Glu Ser Gly Gly Asn Trp Ala Ile Asn Thr Gly
310                 315                 320                 325

aat ggc ttc tcc ggc ggc cta cag ttc cac cca cag acc tgg ctc gca    1363
Asn Gly Phe Ser Gly Gly Leu Gln Phe His Pro Gln Thr Trp Leu Ala
                330                 335                 340

tac ggt ggt gga gct ttc tcc ggt gac gct tcc ggt gca agc cgt gaa    1411
Tyr Gly Gly Gly Ala Phe Ser Gly Asp Ala Ser Gly Ala Ser Arg Glu
            345                 350                 355

cag caa atc tcc atc gca gaa aag gtt cag gct gca caa ggt tgg gga    1459
Gln Gln Ile Ser Ile Ala Glu Lys Val Gln Ala Ala Gln Gly Trp Gly
        360                 365                 370

gca tgg cct gct tgc acc gca agc ttg ggc atc cga tagtagaaat         1505
Ala Trp Pro Ala Cys Thr Ala Ser Leu Gly Ile Arg
    375                 380                 385

ctggcatcca ataggtagat tgggatgcta tggaagaacc ctcaggtgca cagctgctcg 1565

gcccggtaga aatccgtgcg ctggcagaaa agctcgacgt cacaccaact aagaagttgg 1625

ggcagaactt tgttcacgat cccaacacgg tgcgtcgcat tgttgctgcg gcagagctca 1685

ccccagacga ccacgtggtg gaagttggcc ctggtctggg ctctctgacc cttgc      1740

<210> 2
<211> 385
<212> PRT
<213> Corynebacterium glutamicum

<400> 2
Leu Leu Ile Pro Pro Arg Ala Lys Lys Phe Tyr Met Ala Pro His Gln
1               5                   10                  15

Lys Ser Arg Ile Asn Arg Ile Asn Ser Thr Arg Ser Val Pro Leu Arg
            20                  25                  30

Leu Ala Thr Gly Gly Val Leu Ala Thr Leu Leu Ile Gly Gly Val Thr
        35                  40                  45

Ala Ala Ala Thr Lys Lys Asp Ile Ile Val Asp Val Asn Gly Glu Gln
    50                  55                  60

Met Ser Leu Val Thr Met Ser Gly Thr Val Glu Gly Val Leu Ala Gln
65                  70                  75                  80

Ala Gly Val Glu Leu Gly Asp Gln Asp Ile Val Ser Pro Ser Leu Asp
                85                  90                  95

Ser Ser Ile Ser Asp Glu Asp Thr Val Thr Val Arg Thr Ala Lys Gln
            100                 105                 110

Val Ala Leu Val Val Glu Gly Gln Ile Gln Asn Val Thr Thr Thr Ala
        115                 120                 125

Val Ser Val Glu Asp Leu Leu Gln Glu Val Gly Gly Ile Thr Gly Ala
    130                 135                 140

Asp Ala Val Asp Ala Asp Leu Ser Glu Thr Ile Pro Glu Ser Gly Leu
145                 150                 155                 160

Lys Val Ser Val Thr Lys Pro Lys Ile Ile Ser Ile Asn Asp Gly Gly
                165                 170                 175

Lys Val Thr Tyr Val Ser Leu Ala Ala Gln Asn Val Gln Glu Ala Leu
            180                 185                 190

Glu Leu Arg Asp Ile Glu Leu Gly Ala Gln Asp Arg Ile Asn Val Pro
        195                 200                 205

Leu Asp Gln Gln Leu Lys Asn Asn Ala Ala Ile Gln Ile Asp Arg Val
    210                 215                 220

Asp Asn Thr Glu Ile Thr Glu Thr Val Ser Phe Asp Ala Glu Pro Thr
225                 230                 235                 240

Tyr Val Asp Asp Pro Glu Ala Pro Ala Gly Asp Glu Thr Val Val Glu
                245                 250                 255

Glu Gly Ala Pro Gly Thr Lys Glu Val Thr Arg Thr Val Thr Thr Val
            260                 265                 270

Asn Gly Gln Glu Glu Ser Ser Thr Val Ile Asn Glu Val Glu Ile Thr
        275                 280                 285

Ala Ala Lys Pro Ala Thr Ile Ser Arg Gly Thr Lys Thr Val Ala Ala
    290                 295                 300

Asn Ser Val Trp Asp Gln Leu Ala Gln Cys Glu Ser Gly Gly Asn Trp
305                 310                 315                 320

Ala Ile Asn Thr Gly Asn Gly Phe Ser Gly Gly Leu Gln Phe His Pro
                325                 330                 335

Gln Thr Trp Leu Ala Tyr Gly Gly Gly Ala Phe Ser Gly Asp Ala Ser
            340                 345                 350

Gly Ala Ser Arg Glu Gln Gln Ile Ser Ile Ala Glu Lys Val Gln Ala
        355                 360                 365

Ala Gln Gly Trp Gly Ala Trp Pro Ala Cys Thr Ala Ser Leu Gly Ile
    370                 375                 380

Arg
385

<210> 3
<211> 629
<212> DNA
<213> Corynebacterium glutamicum

<400> 3
ggaacttggt gaccaggaca ttgtttcccc ttcactggat tcatccatca gtgatgaaga 60
cactgtgact gttcgtactg ccaagcaggt ggcgctcgtg gtggaaggtc aaatccaaaa 120
cgtgaccacc actgcggttt ccgtggagga cctcctgcag gaagtcggtg gcattaccgg 180
tgctgatgcg gtggacgctg atctttcaga gaccatccca gaatctggtt tgaaggtgag 240
tgttaccaag ccgaagatta tttccatcaa tgatggtggc aaggtcactt acgtttcttt 300
ggcagctcag aacgtacagg aagccctaga gctgcgggat attgagctgg gtgctcagga 360
ccgcattaat gtgcctctgg atcagcagct gaagaacaac gctgcgatcc agatcgaccg 420
cgttgacaac accgaaatca ctgaaactgt gtctttcgat gctgagccaa cctacgtgga 480

tgatccagaa gctccagctg gcgatgaaac tgtggtcgaa gaaggcgctc ctggaaccaa 540
ggaagttact cgcaccgtaa caaccgttaa tggtcaggaa gaatcttcca cggtgatcaa 600
tgaagttgaa atcaccgcag caaagccag 629

Folgende Abbildungen sind beigefügt:
Abbildung 1: Karte des Plasmids pCR2.1zwa2int

Die Längenangaben sind als ca.-Werte zu verstehen.

Die verwendeten Abkürzungen und Bezeichnungen haben folgende Bedeutung.

| | |
|---|---|
| ColE1 ori | Replikationsursprung des Plasmids ColE1 |
| | |
| lacZ | 5'Ende des β-Galactosidase Gens |
| | |
| f1 ori | Replikationsursprung des Phagen f1 |
| | |
| KanR | Kanamycin Resistenz |
| | |
| ApR | Ampicillin Resistenz |
| | |
| EcoRI | Schnittstelle des Restriktionsenzyms EcoRI |
| | |
| zwa2 | internes Fragment des zwa2-Gens |

## Patentansprüche

1. Isoliertes Polynukleotid enthaltend eine Polynukleotidsequenz, ausgewählt aus der Gruppe
a) Polynukleotid, das mindestens zu 70 % identisch ist mit einem Polynukleotid, das für ein Polypeptid codiert, das die Aminosäuresequenz von SEQ ID NO 2 enthält,
b) Polynukleotid, das für ein Polypeptid codiert, das eine Aminosäuresequenz enthält, die zu mindestens 70 % identisch ist mit der Aminosäuresequenz von SEQ ID NO 2,
c) Polynukleotid, das komplementär ist zu den Polynukleotiden von a) oder b), und
d) Polynukleotid, enthaltend mindestens 15 aufeinanderfolgende Nukleotide der Polynukleotidsequenz von a), b) oder c).

2. Polynukleotide gemäß Anspruch 1,
wobei das Polynukleotid eine in coryneformen Bakterien replizierbare, bevorzugt rekombinante DNA ist.

3. Polynukleotide gemäß Anspruch 1,
wobei das Polynukleotid eine RNA ist.

4. Polynukleotide gemäß Anspruch 2,
enthaltend die Nukleinsäuresequenz wie in SEQ ID NO 1 dargestellt.

5. Replizierbare DNA gemäß Anspruch 2 enthaltend
(i) die Nukleotidsequenz, gezeigt in SEQ ID NO 1, oder
(ii) mindestens eine Sequenz, die den Sequenzen (i) innerhalb des Bereichs der Degeneration des genetischen Codes entspricht, oder
(iii) mindestens eine Sequenz, die mit den zur den Sequenzen (i) oder (ii) komplementären Sequenzen hybridisiert, und gegebenenfalls
(iv) funktionsneutrale Sinnmutationen in (i).

6. Vektor, insbesondere Pendelvektor (shuttle vektor) pCR2.1zwa2int,
**gekennzeichnet**
durch die in der Abb.1 wiedergegebenen Restriktionskarte und hinterlegt unter der Bezeichnung DSM 13113 in E.coli DH5α.

7. Coryneforme Bakterien erhalten durch Integrationsmutagenese mit dem Vektor gemäss Anspruch 6.

8. Verfahren zur Herstellung von L-Aminosäuren, insbesondere L-Lysin,
**dadurch gekennzeichnet,**
daß man folgende Schritte durchführt,
a) Fermentation der die gewünschte L-Aminosäure produzierenden Bakterien, in denen man zumindest das zwa2-Gen abschwächt,
b) Anreicherung des gewünschten Produkts im Medium oder in den Zellen der Bakterien, und
c) Isolieren der L-Aminosäure.

9. Verfahren gemäß Anspruch 8,
**dadurch gekennzeichnet,**
daß man Bakterien einsetzt, in denen man zusätzlich weitere Gene des Biosyntheseweges der gewünschten L-Aminosäure, insbesondere das zwa1-Gen, verstärkt.

10. Verfahren gemäß Anspruch 8,
**dadurch gekennzeichnet,**
daß man Bakterien einsetzt, in denen die Stoffwechselwege zumindest teilweise ausgeschaltet sind, die die Bildung der gewünschten L-Aminosäure verringern.

11. Verfahren gemäß Anspruch 8,
**dadurch gekennzeichnet,**
daß man die Expression des Polynukleotids, das für das zwa2-Gen codiert, verringert.

12. Verfahren gemäß Anspruch 8,
**dadurch gekennzeichnet,**
daß man die katalytischen Eigenschaften des Polypeptids (Enzymproteins) herabsetzt, für das das Polynukleotid zwa2 codiert.

13. Verfahren gemäß Anspruch 8,
**dadurch gekennzeichnet,**
daß man zur Erzielung der Abschwächung das Verfahren der Integrationsmutagenese mittels des Vektors pCR2.1zwa2int, dargestellt in Figur 1 und hinterlegt in E.coli als DSM 13113, verwendet.

14. Verfahren gemäß Anspruch 8,
**dadurch gekennzeichnet,**
daß man für die Herstellung von L-Lysin Bakterien fermentiert, in denen man gleichzeitig eines oder mehrere der Gene, ausgewählt aus der Gruppe
14.1 das für die Dihydrodipicolinat-Synthase kodierende dapA-Gen,
14.2 das für eine feed back resistente Aspartatkinase kodierende lysC-Gen,
14.3 das für die Pyruvat-Carboxylase kodierende pyc-Gen,
14.4 das für die Tetradihydrodipicolinat Succinylase kodierende dapD-Gen,
14.5 das Gen für die Succinyldiaminopimelate-Desuccinylase kodierende dapE-Gen,
14.6 das für die Glyceraldehyd-3-Phosphat Dehydrogenase kodierende gap-Gen,
14.7 das für die Malat:Chinon Oxidoreduktase kodierende mqo-Gen,
14.8 das für den Lysin-Export kodierende lysE-Gen,
verstärkt, insbesondere überexprimiert oder amplifiziert.

15. Verfahren gemäß Anspruch 8,
**dadurch gekennzeichnet,**
daß man für die Herstellung von L-Lysin Bakterien fermentiert, in denen man gleichzeitig eines oder mehrere der Gene, ausgewählt aus der Gruppe
15.1 das für die Phosphoenolpyruvat-Carboxykinase codierende pck-Gen,
15.2 das für die Glucose-6-Phosphat Isomerase kodierende pgi-Gen
abschwächt.

16. Verfahren gemäß einem oder mehreren der vorhergehenden Ansprüche,
**dadurch gekennzeichnet,**
daß man Mikroorganismen der Gattung Corynebacterium glutamicum einsetzt.

17. Verwendung von Polynukleotidsequenzen gemäß Anspruch 1 oder Teile davon
als Hybridisierungssonden zur Isolierung von cDNA, die für das Zwa2-Genprodukt codiert.

18. Verwendung von Polynukleotidsequenzen gemäß Anspruch 1 oder Teilen davon
als Hybridisierungssonden zur Isolierung von cDNA oder Genen, die eine hohe Ähnlichkeit mit der Sequenz des Zwa2-Gens aufweisen.
